# EUROPEAN PATENT SPECIFICATION

(11) **EP 2 499 260 B1**
(45) Date of publication and mention of the grant of the patent: **23.08.2017**
(21) Application number: 10830319.9
(22) Date of filing: 09.11.2010
(51) Int. Cl.: C12Q 1/68, G01N 33/53

(54) **HEART AGING BIOMARKERS AND METHODS OF USE**
HERZALTERUNGS-BIOMARKER UND VERFAHREN ZU IHRER VERWENDUNG
BIOMARQUEURS DE VIEILLISSEMENT DU COEUR ET LEURS PROCÉDÉS D'UTILISATION

(30) Priority: 10.11.2009 US 280879 P
(43) Date of publication of application: 19.09.2012
(73) Proprietor: Nestec S.A., 1800 Vevey (CH)
(72) Inventor: LI, Qinghong, Chesterfield, MO 63305 (US)
(74) Representative: Rupp, Christian
(86) International application number: PCT/US2010/002950
(87) International publication number: WO 2011/059490

(56) References cited:
- US-A1- 2005 136 537
- US-A1- 2007 072 208
- US-A1- 2007 196 850
- US-A1- 2008 254 464
- ASHTON K J ET AL: "Age-associated shifts in cardiac gene transcription and transcriptional responses to ischemic stress", EXPERIMENTAL GERONTOLOGY, vol. 41, no. 2, 1 February 2006 (2006-02-01), pages 189-204, XP027937001, ELSEVIER SCIENCE, OXFORD, GB ISSN: 0531-5565 [retrieved on 2006-02-01]
- BODYAK N ET AL: "Gene expression profiling of the aging mouse cardiac myocytes", NUCLEIC ACIDS RESEARCH, vol. 30, no. 17, 1 September 2002 (2002-09-01), pages 3788-3794, XP002341760, OXFORD UNIVERSITY PRESS, SURREY, GB ISSN: 0305-1048, DOI: 10.1093/NAR/GKF497
- LIU H ET AL.: "Augmented Wnt signaling in a mammalian model of accelerated aging", SCIENCE, vol. 317, 10 August 2007 (2007-08-10), pages 803-806, XP002698336,
- BARGER J L ET AL.: "A low dose of dietary resveratrol partially mimics caloric restriction and reteards aging parameters in mice", PLOS ONE, vol. 3, no. 6, E2264, June 2008 (2008-06), pages 1-10, XP002698337,
- MAIESE ET AL.: 'The WNT signaling pathway: Aging gracefully as a protectionist?' PHARMACOLOGY & THERAPEUTICS vol. 118, no. 1, April 2008, pages 58 - 81, XP008156478
- 'Wnt (Pathway) from Homo sapiens' CANCER CELL MAP DATABASE FROM MEMORIAL SLOAN-KETTERING CANCER CENTER AND THE INSTITUTE OF BIOINFORMATICS, [Online] 2006, XP008160083 Retrieved from the Internet: <URL:http://cancer.cellmap.org/cellmap/reco rd.do?id=12368show_flags=010> [retrieved on 2010-12-31]

## Description

### CROSS REFERENCE TO RELATED APPLICATIONS

This application claims priority to U.S. Provisional Application Serial No. 61/280879 filed November 10, 2009.

### BACKGROUND OF THE INVENTION

### Field of the Invention

The invention relates generally to the field of nutritional support of health and longevity in animals. In particular, the invention provides a set of biomarkers of aging in the heart, comprising several genes involved in the Wnt signaling pathway. The invention further provides for the use of those biomarkers to identify nutrients and dietary regimens for retarding heart aging, and for modulation of the genes themselves to retard heart aging.

### Description of Related Art

Aging of an organism and its component organs is currently the subject of much attention in biological and medicinal research. Aging hallmarks include decreased stem and progenitor cell function and a decline in tissue regeneration (Rando, 2006, Nature 441: 1080-1086). Aged tissues exhibit reduced intrinsic resistance to injury or damage. Resident and circulating stem and progenitor cells are unspecialized cells that are critical for ongoing repairs of injured tissues. This regenerative function diminishes as the tissue ages. It has been postulated that the stem and progenitor cell dysfunction may contribute to aging and disease.

Recent reports have described a connection between the Wnt/beta-catenin signaling pathway and stem cell aging (*see, e.g.,* White et al., 2007, Current Biology 17(21): R923-925; Brack et al., 2007, Science 317: 807-810; Liu et al., 2007, Science 317: 803-806; Nusse, 2008, Cell Research 18: 523-527; Rando, 2006, *supra*). Beta-catenin is a key mediator of the Wnt signaling pathway (Willert & Nusse, 1998, Current Opinion in Genetics & Development 8: 95-102; Nusse, 2008, *supra*). When a Wnt protein binds to its receptors on the cell membrane, a chain of biochemical reactions is triggered inside the cell. The signaling cascade is eventually passed into the nucleus *via* beta-catenin, which relays the Wnt signal from cytosol to nucleus to regulate gene expression with other nuclear proteins (Huelsken & Birchmeirer, 2001, Curr. Op. Genet. & Devel. 11:547-553).

It has been reported that Wnt signaling increased in various tissues and organs of animal models of accelerated aging (Liu *et al.,* 2007, *supra*). Continuous Wnt exposure was reported to cause cellular senescence. Similarly, increased Wnt signaling in aged skeletal muscle stem cells has been reported (Brack *et al.,* 2007, *supra*). Further, Wnt signaling was reported to promote myogenic-to-fibrogenic conversion of muscle stem cells and inhibition of this signal preserved myogenic fate.

In heart, Deb *et al.* reported that antagonizing Wnt signaling with the secreted frizzled related protein 2 (SFRP2), an extracellular Wnt antagonist, inhibited differentiation and maintained embryonic and adult cardiac progenitors in an undifferentiated state (Deb et al., 2008, Stem Cells 26: 35-44). Those researchers therefore hypothesized that Wnt signaling increases with aging and this signaling drives cardiac stem and progenitor cells into fibrogenic lineage (Deb, 2008, Research Proposal Summary - New Scholar Award in Aging, Ellison Medical Foundation at URL ellisonfoundation.org/). Ashton *et al.* reported down-regulation of the Wnt signaling pathway in "aged" versus young heart. (Ashton et al., 2005, Experimental Gerontology 41: 189-204), but the expression of beta-catenin was not reported in that study. Moreover, even though it has been shown that functionally competent cardiac progenitor cells peak at the age of 20 months in mice (Gonzales et al., 2008, Circulation Research 102: 597-606), the mice in the "aged" group of the Ashton *et al.* study ranged in age from 16-18 months old. Thus, the mice used in the Ashton *et al.* study were not truly aged individuals.

Restriction of caloric intake well below *ad libitum* levels has been shown to increase lifespan, reduce or delay the onset of many age-related conditions, improve stress resistance and decelerate functional decline in numerous animal species, including mammals such as rodents and primates. Indeed, clinical trials have been initiated to evaluate the longevity-promoting effect of caloric restriction (CR) in humans. But in humans and animals alike, it seems unlikely that CR is a viable strategy for increasing longevity in most individuals, due to the degree and length of restriction required. For this reason, research has focused on the identification of substances, e.g., pharmaceutical agents, nutritional substances and the like, capable of mimicking the effect of CR without a substantive change in dietary intake.

Efforts have been directed toward identifying agents that can mimic one or more of the physiological or biochemical effects of CR (*e.g.,* Barger et al., 2008, PLoS ONE 3(6): e2264. doi:10.1371/journal.pone.0002264), or that can mimic the gene expression profile associated with CR in certain tissues and organs (e.g., Spindler, U.S. Patent 6,406,853; U.S. Patent Pub. 2003/0124540; Barger *et al.,* 2008, *supra*). In connection with the latter, methods to analyze genes associated with CR and to screen for CR mimetics based on gene expression profiling have been disclosed (Spindler *et al.,* U.S. Patent Pubs. 2004/0180003, 2004/0191775 and 2005/0013776; Pan et al., U.S. Patent Pub. 2007/0231371).

A number of substances have been reported to interact with beta-catenin and/or Wnt signaling in a variety of non-cardiac cell and tissue systems, including: alginate (Dettmar et al., 2007, Biomater Sci Polym Ed 18: 317-333), apigenin Shukla et al., 2007, Cancer Res. 67: 6925-6935), ascorbic acid (Quintanilla et al., 2005, J Biol Chem 280:1 1615-11625), curcumin (Mahmoud et al., 2000, Carcinogenesis 21: 921-927), flavanone (Park et al., 2005, Biochem Biophys Res Commun 331: 1222-1228), genistein (Guo et al., 2002, Am J Physiol Cell Physiol 283: C722-C734), hyaluronic acid (Bourguignon et al., 2007, JBiol Chem 282: 1265-1280), magnesium (Caveda et al., 1996, J Clin Invest 98: 886-893), monooleyl phosphatidic acid (Malbon, 2005, Sci STKE 292: pe35), naringenin (Lee et al., 2005, Biochem Biophys Res Commun 335: 771-776), nitric oxide (nitroglycerin) (Prévotat et al., 2006, Gastroenterology 131: 1142-1152), quercetin (van Erk et al., 2005, Eur JNutr 44: 143-556), resveratrol (Hope et al., 2008, Mol Nutr Food Res 52: S52-S61), S-carbamylcysteine (Proweller et al., 2006, Cancer Res 66: 7438-7444), sodium butyrate (Abramova et al., 2006, J Biol Chem 281: 21040-21051), sodium salicylate (Lee et al., 2003, Int J Oncol 23: 503-508), spermidine (Guo et al., 2002, Am J Physiol Cell Physiol 283: C722-734), sphingosine (Olsson et al., 2004, Am J Physiol Gastrointest Liver Physiol 287: G929-937), trolox (a vitamin E derivative) *(Quintanilla et al.,* 2005, *supra*), and glucose (Lin et al., 2006, JAm Soc Nephrol 17: 2812-2820). However, the mechanisms by which such interactions were reported to occur, and the effect of such interactions, were not well understood in many cases.

Ashton *et al.* reported some age associated shifts in cardiac gene transcription and transcriptional responses to ischemic stress. In this light it was reported that aging is associated with shifts in cardiovascular gene expression consistent with the phenotypic features of older hearts. It was further reported that reduced tolerance with age may be related to the modification of signaling (particularly WNT and TGF-β), and shifts in expression of immediate early genes, and genes important in control of cell death/survival, angiogenesis, and cardiac remodelling (Ashton et al., 2006, Experimental Gerontology 41: 189-204).

Bodyak *et al.* reported some gene expression profiling of the aging mouse cardiac myocytes. In this light Bodyak *et al.* reported that genes whose mRNA levels change with aging in cardiomyocytes might profoundly affect pathological changes in the heart (Bodyak et al., 2002, Nucleic Acids Research 30: 3788-3794).

Despite the availability of the approaches summarized above, there remains a need for more robust, faster, and less costly methods to screen for agents that can retard or reverse the aging process, either of the whole body or of specific tissues and organs, to promote healthy aging and increase longevity. The present invention satisfies these needs.

### SUMMARY OF THE INVENTION

It is therefore an object of the invention to provide methods for measuring biological aging of heart cells or tissue.

It is another object of the invention to provide a method for measuring the effect of a test substance on the expression profile of one or more genes differentially expressed in heart cells and tissues of aged subjects, as compared with young subjects or a standard reference.
One or more of these other objects are achieved using novel combinations of polynucleotides or polypeptides representing genes and gene segments that are differentially expressed in heart cells and tissues of aged subjects as compared with young subjects, wherein the genes are related by their involvement in the wnt signaling pathway in the heart, by the decrease in their expression in aged versus young heart tissue, and by the reversal of such age-related decreases by cr or by ingestion of resveratrol. The polynucleotides are used to produce methods for determining the status of polynucleotides differentially expressed in selected tissues of aged subjects, as compared with young subjects or a standard reference, which are useful for achieving the above-identified objects, *e.g.,* for screening substances to determine if they are likely to have an anti-aging effect in the heart.

The invention is directed to a method for screening an agent or regimen for the ability to retard heart aging comprising the steps of:
a) determining a first gene expression profile by measuring transcription or translation products of one or more genes related to Wnt signaling in heart tissue from an aged subject in the absence of the agent or regimen;
b) determining a second gene expression profile by measuring transcription or translation products of one or more genes related to Wnt signaling in heart tissue from an aged subject in the presence of the agent or regimen; and
c) comparing the first gene expression profile with the second gene expression profile, wherein a change in the second gene expression profile indicates that the material or regimen is likely to be useful in retarding heart aging when administered to an individual.

The method can further comprise comparing at least the second gene expression profile with a reference gene expression profile obtained by measuring transcription or translation products of one or more genes related to Wnt signaling in heart tissue from a young subject or in heart tissue from an aged subject in the presence of a reference substance or regimen known to retard heart aging when administered to an individual; optionally wherein the reference substance or regimen comprises caloric restriction or ingestion of resveratrol.

The method can comprise wherein the genes are Dlgh1, Magi3, Akt1, Dab2, Rac1, Ctnnb1, Camk2d, Mapk1, Senp2, Smad3, Mark2, Ccnd1 or Pias4.

The method can comprise wherein the genes are Dlgh1, Magi3, Ctnnb1, Camk2d, Mapk1 or Senp2.

The method can comprise wherein the genes are Magi3, Ctnnb1 or Camk2d.

The method can comprise wherein the gene is Ctnnb1.

### DETAILED DESCRIPTION OF THE INVENTION

### Definitions

The term "aging" means biological or physiological aging of an organism, organ, tissue, or any portion thereof. This includes the development of age-related diseases in an organism, organ, tissue, or any portion thereof. Similarly "heart aging" means biological or physiological aging of the heart or any portion thereof, including development of age-related diseases in the heart or any portion thereof. Reference to the heart or any other organ referred to herein is intended to include all cells and tissues comprising the heart or other organ.

The term "animal" means a human or other animal, including avian, bovine, canine, equine, feline, hicrine, murine, ovine, and porcine animals. When the term is used in the context of comparing test subjects, the animals that are compared are animals of the same species and possibly of the same race or breed. The term "non-human animal" may be used herein to refer to all animals except humans. A "companion animal" is any domesticated animal, and includes, without limitation, cats, dogs, rabbits, guinea pigs, ferrets, hamsters, mice, gerbils, horses, cows, goats, sheep, donkeys, pigs, and the like. In particular, the animal is a human or a companion animal, such as a dog or cat.

The term "antibody" means any immunoglobulin that binds to a specific antigen, including IgG, IgM, IgA, IgD, and IgE antibodies. The term includes polyclonal, monoclonal, monovalent, humanized, heteroconjugate, antibody compositions with polyepitopic specificity, chimeric, bispecific antibodies, diabodies, single-chain antibodies, and antibody fragments such as Fab, Fab', F(ab')2, and Fv, or other antigen-binding fragments.

The term "array" means an ordered arrangement of at least two probes on a substrate. At least one of the probes is a control or standard and at least one of the probes is a diagnostic probe. The arrangement of from about two to about 40,000 probes on a substrate assures that the size and signal intensity of each labeled complex formed between a probe and a sample polynucleotide or polypeptide is individually distinguishable.

The term "binding complex" refers to a complex formed when a polypeptide in a sample specifically binds (as defined herein) to a binding partner, such as an antibody or functional fragment thereof.

"Calorie restriction" or "caloric restriction" ("CR") refer to any diet regimen low in calories without undernutrition. In general, the limitation is of total calories derived from of carbohydrates, fats, and proteins. The limitation is typically, although not limited to, about 25% to about 40% of the caloric intake relative to ad libitum consumption.

A "dietary supplement" is a product that is intended to be ingested in addition to the normal diet of an animal. Dietary supplements may be in any form, *e.g.,* solid, liquid, gel, tablets, capsules, powder, and the like. Preferably they are provided in convenient dosage forms. Disclosed is that they are provided in bulk consumer packages such as bulk powders or liquids. Disclosed is that supplements are provided in bulk quantities to be included in other food items such as snacks, treats, supplement bars, beverages and the like.

The term "effective amount" means an amount of a compound, material, composition, medicament, or other material, or to the conditions of a regimen, such as a dietary or exercise regimen, that is effective to achieve a particular biological result, such as reversing or retarding aging in a selected tissue such as the heart, as described herein.

The term "expression", e.g., "gene expression" means the transcription of a gene to produce mRNA, and the translation of the mRNA to produce a protein. An increase in the rate of transcription or translation, or an increase in the production of a product of transcription or translation, e.g., mRNA or protein, is included in the terms "increased gene expression," "up-regulated gene expression," and the like. Similarly, a decrease in the rate of transcription or translation, or a decrease in the production of a product of transcription or translation, *e.g.,* mRNA or protein, is included in the terms "decreased gene expression," "down-regulated gene expression," and the like. The terms "modulation of gene expression," "affecting gene expression," and the like, refer to increasing or decreasing gene expression. The term "differential expression" or "differentially expressed" means increased or up-regulated gene expression, or decreased or down-regulated gene expression, as detected by the absence, presence, or at least 1.2-fold change in the amount of transcribed messenger RNA or translated protein in a sample.

"Extended" administration as used herein generally refers to periods in excess of one month. Periods of longer than two, three, or four months are contemplated. Also included are more extended periods that include longer than 5, 6, 7, 8, 9, or 10 months. Periods in excess of 11 months or 1 year are also included. Longer terms use extending over 1, 2, 3, or more years are also contemplated herein. In the case of certain animals, it is envisioned that the animal would be administered substances identified by the present methods on a regular basis. "Regular basis," "regular administration" and/or "regular ingestion" as used herein refers to at least weekly, administration. More frequent administration, such as twice or thrice weekly is contemplated. Also included are regimens that comprise at least once, twice, three times or more daily administration. Any dosing frequency, regardless of whether expressly exemplified herein, is considered useful. The skilled artisan will appreciate that dosing frequency will be a function of the substance that is being administered, and some compositions may require more or less frequent administration to maintain a desired biochemical, physiological or gene expression effects, namely effects including one or more of food intake, satiety, lipid metabolism, and fat utilization, and the gene expression profile associated therewith. The term "extended regular basis," "extended regular administration," and/or "extended regular ingestion," as used herein, refers to extended administration of a substance on a regular basis.

The term "food" or "food composition" means a composition that is intended for consumption by an animal, including a human, and provides nutrition thereto. A "food product formulated for human consumption" is any composition specifically intended for ingestion by a human being. "Pet foods" are compositions intended for consumption by pets, preferably by companion animals. A "complete and nutritionally balanced pet food," is one that contains all known required nutrients for the intended recipient or consumer of the food, in appropriate amounts and proportions, based for example on recommendations of recognized authorities in the field of companion animal nutrition. Such foods are therefore capable of serving as a sole source of dietary intake to maintain life or promote production, without the addition of supplemental nutritional sources. Nutritionally balanced pet food compositions are widely known and widely used in the art.

The term "fragment" means (1) an oligonucleotide or polynucleotide sequence that is a portion of a complete sequence and that has the same or similar activity for a particular use as the complete polynucleotide sequence or (2) a peptide or polypeptide sequence that is a portion of a complete sequence and that has the same or similar activity for a particular use as the complete polypeptide sequence. Such fragments can comprise any number of nucleotides or amino acids deemed suitable for a particular use. Generally, oligonucleotide or polynucleotide fragments contain at least about 10, 50, 100, or 1000 nucleotides and polypeptide fragments contain at least about 4, 10, 20, or 50 consecutive amino acids from the complete sequence. The term encompasses polynucleotides and polypeptides variants of the fragments.

The term "gene" or "genes" means a complete or partial segment of DNA involved in producing a polypeptide, including regions preceding and following the coding region (leader and trailer) and intervening sequences (introns) between individual coding segments (exons). The term encompasses any DNA sequence that hybridizes to the complement of gene coding sequences.

The term "gene product" means the product of transcription of a gene, such as mRNA or derivatives thereof (e.g., cDNA), or translation of a gene transcript. The term "gene product" generally refers to the translation product, which is a protein. The term "gene product" may be used interchangeably with the term "protein" or "polypeptide" herein.

The term "homolog" means (1) a polynucleotide, including polynucleotides from the same or different animal species, having greater than 30%, 50%, 70%, or 90% sequence similarity to a reference polynucleotide, and having the same or substantially the same properties and performing the same or substantially the same function as the reference polynucleotide, or having the capability of specifically hybridizing to a reference polynucleotide under stringent conditions or (2) a polypeptide, including polypeptides from the same or different animal species, having greater than 30%, 50%, 70%, or 90% sequence similarity to a reference polypeptide and having the same or substantially the same properties and performing the same or substantially the same function as the reference polypeptide, or having the capability of specifically binding to a reference polypeptide. When referring to fragments of full length coding sequences, the function of those fragments may simply be to encode a selected portion of a polypeptide of a certain sequence, or to be of suitably similar sequence to hybridize to another polynucleotide fragment encoding that polypeptide. When referring to fragments of polypeptides, the function of those fragments may simply be to form an epitope suitable for generation of an antibody. Sequence similarity of two polypeptide sequences or of two polynucleotide sequences is determined using methods known to skilled artisans, *e.g*., the algorithm of Karlin and Altschul (Proc. Natl. Acad. Sci. USA 87:2264-2268 (1990)). Such an algorithm is incorporated into the NBLAST and XBLAST programs of Altschul et al., 1990, (J. Mol. Biol. 215:403-410). To obtain gapped alignments for comparison purposes, Gapped Blast can be utilized as described in Altschul et al., 1997, (Nucl. Acids Res. 25: 3389-3402). When utilizing BLAST and Gapped BLAST programs, the default parameters of the respective programs (*e.g*., XBLAST and NBLAST) are used.

The term "hybridization complex" means a complex that is formed between sample polynucleotides when the purines of one polynucleotide hydrogen bond with the pyrimidines of the complementary polynucleotide, *e.g*., 5'-A-G-T-C-3' base pairs with 3'-T-C-A-G-5'. The degree of complementarily and the use of nucleotide analogs affect the efficiency and stringency of hybridization reactions.

The term "in conjunction" means that a drug, food, or other substance is administered to an animal (1) together in a composition, particularly food composition, or (2) separately at the same or different frequency using the same or different administration routes at about the same time or periodically. "Periodically" means that the substance is administered on a dosage schedule acceptable for a specific substance. "About the same time" generally means that the substance (food or drug) is administered at the same time or within about 72 hours of each other. "In conjunction" specifically includes administration schemes wherein substances such as drugs are administered for a prescribed period and compositions of the invention are administered indefinitely.

The term "individual" when referring to an animal means an individual animal of any species or kind. This term is used interchangeably with the term "subject."

The term "polynucleotide" or "oligonucleotide" means a polymer of nucleotides. The term encompasses DNA and RNA (including cDNA and mRNA) molecules, either single or double stranded and, if single stranded, its complementary sequence in either linear or circular form. The term also encompasses fragments, variants, homologs, and alleles, as appropriate for the sequences, which have the same or substantially the same properties and perform the same or substantially the same function as the original sequence. In particular, the term encompasses homologs from different species, *e.g*., a mouse and a dog or cat. The sequences may be fully complementary (no mismatches) when aligned or may have up to about a 30% sequence mismatch. Optionally, for polynucleotides, the chain contains from about 50 to 10,000 nucleotides, more preferably from about 150 to 3,500 nucleotides. Optionally, for oligonucleotides, the chain contains from about 2 to 100 nucleotides, more preferably from about 6 to 30 nucleotides. The exact size of a polynucleotide or oligonucleotide will depend on various factors and on the particular application and use of the polynucleotide or oligonucleotide. The term includes nucleotide polymers that are synthesized and that are isolated and purified from natural sources. The term "polynucleotide" is inclusive of "oligonucleotide."

The term "polypeptide," "peptide," or "protein" means a polymer of amino acids. The term encompasses naturally occurring and non-naturally occurring (synthetic) polymers and polymers in which artificial chemical mimetics are substituted for one or more amino acids. The term also encompasses fragments, variants, and homologs that have the same or substantially the same properties and perform the same or substantially the same function as the original sequence. The term encompass polymers of any length, optionally polymers containing from about 2 to 1000 amino acids, more specifically from about 5 to 500 amino acids. The term includes amino acid polymers that are synthesized and that are isolated and purified from natural sources.

The term "probe" means (1) an oligonucleotide or polynucleotide, either RNA or DNA, whether occurring naturally as in a purified restriction enzyme digest or produced synthetically, that is capable of annealing with or specifically hybridizing to a polynucleotide with sequences complementary to the probe or (2) a compound or substance, including a peptide or polypeptide, capable of specifically binding a particular protein or protein fragment to the substantial exclusion of other proteins or protein fragments. An oligonucleotide or polynucleotide probe may be either single or double stranded. The exact length of the probe will depend upon many factors, including temperature, source, and use. For example, for diagnostic applications, depending on the complexity of the target sequence, an oligonucleotide probe typically contains about 10 to 100, 15 to 50, or 15 to 25 nucleotides. In certain diagnostic applications, a polynucleotide probe contains about 100-1000, 300-600, nucleotides, preferably about 300 nucleotides. The probes herein are selected to be "substantially" complementary to different strands of a particular target sequence. This means that the probes must be sufficiently complementary to specifically hybridize or anneal with their respective target sequences under a set of predetermined conditions. Therefore, the probe sequence need not reflect the exact complementary sequence of the target. For example, a noncomplementary nucleotide fragment may be attached to the 5' or 3' end of the probe, with the remainder of the probe sequence being complementary to the target sequence. Alternatively, noncomplementary bases or longer sequences can be interspersed into the probe if the probe sequence has sufficient complementarity with the sequence of the target polynucleotide to specifically anneal to the target polynucleotide. A peptide or polypeptide probe may be any molecule to which the protein or peptide specifically binds, including DNA (for DNA binding proteins), antibodies, cell membrane receptors, peptides, cofactors, lectins, sugars, polysaccharides, cells, cell membranes, organelles and organellar membranes.

The term "sample" means any animal tissue or fluid containing, *e.g*., polynucleotides, polypeptides, antibodies, metabolites, and the like, including cells and other tissue containing DNA and RNA. Examples include adipose, blood, cartilage, connective, epithelial, lymphoid, muscle, nervous, sputum, and the like. A sample may be solid or liquid and may be DNA, RNA, cDNA, bodily fluids such as blood or urine, cells, cell preparations or soluble fractions or media aliquots thereof, chromosomes, organelles, and the like.

The term "single package" means that the components of a kit are physically associated in or with one or more containers and considered a unit for manufacture, distribution, sale, or use. Containers include, but are not limited to, bags, boxes, bottles, shrink wrap packages, stapled or otherwise affixed components, or combinations thereof. A single package may be containers of individual food compositions physically associated such that they are considered a unit for manufacture, distribution, sale, or use.

The term "specifically bind" means a special and precise interaction between two molecules which is dependent upon their structure, particularly their molecular side groups. For example, the intercalation of a regulatory protein into the major groove of a DNA molecule, the hydrogen bonding along the backbone between two single stranded nucleic acids, or the binding between an epitope of a protein and an agonist, antagonist, or antibody.

The term "specifically hybridize" means an association between two single stranded polynucleotides of sufficiently complementary sequence to permit such hybridization under predetermined conditions generally used in the art (sometimes termed "substantially complementary"). For example, the term may refer to hybridization of a polynucleotide probe with a substantially complementary sequence contained within a single stranded DNA or RNA molecule according to an aspect of the invention, to the substantial exclusion of hybridization of the polynucleotide probe with single stranded polynucleotides of non-complementary sequence.

The term "standard" or "reference" means (1) a control sample that contains tissue from an individual administered a control or reference substance, or no substance, as compared with a sample that contains tissue from an individual administered a test substance, for example, to determine if the test substance causes differential gene expression, as appropriate for the context of its use.

The term "stringent conditions" means (1) hybridization in 50% (vol/vol) formamide with 0.1% bovine serum albumin, 0.1% Ficoll, 0.1% polyvinylpyrrolidone, 50 mM sodium phosphate buffer at pH 6.5 with 750 mM NaCl, 75 mM sodium citrate at 42°C, (2) hybridization in 50% formamide, 5x SSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42°C; with washes at 42°C in 0.2x SSC and 0.1% SDS or washes with 0.015 M NaCl, 0.0015 M sodium citrate, 0.1% Na2SO4 at 50°C or similar procedures employing similar low ionic strength and high temperature washing agents and similar denaturing agents.

The term "variant" means (1) a polynucleotide sequence containing any substitution, variation, modification, replacement, deletion, or addition of one or more nucleotides from or to a polynucleotide sequence and that has the same or substantially the same properties and performs the same or substantially the same function as the original sequence and (2) a polypeptide sequence containing any substitution, variation, modification, replacement, deletion, or addition of one or more amino acids from or to a polypeptide sequence and that has the same or substantially the same properties and performs the same or substantially the same function as the original sequence. The term therefore includes single nucleotide polymorphisms (SNPs) and allelic variants and includes conservative and non-conservative amino acid substitutions in polypeptides. The term also encompasses chemical derivatization of a polynucleotide or polypeptide and substitution of nucleotides or amino acids with nucleotides or amino acids that do not occur naturally, as appropriate.

The term "virtual package" means that the components of a kit are associated by directions on one or more physical or virtual kit components instructing the user how to obtain the other components, *e.g.,* in a bag containing one component and directions instructing the user to go to a website, contact a recorded message, view a visual message, or contact a caregiver or instructor to obtain instructions on how to use the kit.

The terms "Wnt signaling," "Wnt pathway," and the like refer to a well known signal transduction pathway that involves a complex network of genes and their encoded proteins involved in embryogenesis, as well as in normal physiological processes in adult animals. These genes and proteins, sometimes referred to herein as "Wnt-related" genes or proteins, produce or regulate the production of Wnt signaling molecules, their interactions with receptors on target cells and the physiological responses of target cells that result from the exposure of cells to the extracellular Wnt ligands.

"Young" refers generally to an individual in young adulthood, *i.e.,* matured past puberty or adolescence, as would be defined by species, or by strain, breed or ethnic group within a species, in accordance with known parameters. "Aged" or "old," as used herein, refers to an individual who is physically or chronologically within the last 30% of its average life expectancy, as determined by species, or by strain, breed or ethnic group within a species, in accordance with known parameters.

Ranges are used herein as shorthand to avoid having to list and describe each and every value within the range. Any appropriate value within the range can be selected, where appropriate, as the upper value, lower value, or the terminus of the range.

As used herein, the singular form of a word includes the plural, and vice versa, unless the context clearly dictates otherwise. Thus, the references "a", "an", and "the" are generally inclusive of the plurals of the respective terms. For example, reference to "an individual," "an animal", "a method", or "a disease" includes a plurality of such "subjects" "animals", "methods", or "diseases." Similarly, the words "comprise", "comprises", and "comprising" are to be interpreted inclusively rather than exclusively. Likewise the terms "include", "including" and "or" should all be construed to be inclusive, unless such a construction is clearly prohibited from the context. Similarly, the term "examples," particularly when followed by a listing of terms, is merely exemplary and illustrative and should not be deemed to be exclusive or comprehensive.

The term "comprising" is intended to include embodiments encompassed by the terms "consisting essentially of" and "consisting of". Similarly, the term "consisting essentially of" is intended to include embodiments encompassed by the term "consisting of".

Unless defined otherwise, all technical and scientific terms, terms of art, and acronyms used herein have the meanings commonly understood by one of ordinary skill in the art in the field(s) of the invention, or in the field(s) where the term is used. Although any compositions, methods, articles of manufacture, or other means or materials similar or equivalent to those described herein can be used in the practice of the invention, the preferred compositions, methods, articles of manufacture, or other means or materials are described herein.

All patents, patent applications, publications, technical and/or scholarly articles, and other references cited or referred to herein, the discussion of those references is intended merely to summarize the assertions made therein. No admission is made that any such patents, patent applications, publications or references, or any portion thereof, are relevant, material, or prior art. The right to challenge the accuracy and pertinence of any assertion of such patents, patent applications, publications, and other references as relevant, material, or prior art is specifically reserved.

### The Invention

The many aspects of the invention described herein arise in part from the inventors' discovery of an age-related decrease in expression of several genes that are positive determinants of the Wnt signaling pathway in the heart. The observation was made in two different animal species, indicating that the observed down-regulation of the Wnt pathway in aged heart is not species-specific. Notably, beta-catenin, an important player in Wnt signaling, is also down-regulated. These observations contrasts with many of the past and recent findings that Wnt signaling increases with aging. The inventors also found that nutritional interventions, such as calorie restriction (CR) and resveratrol supplementation, known to retard the aging process in general, suppressed the aging-related decrease in Wnt pathway gene expression, and increased the expression of many of the Wnt genes back to levels observed in heart tissue from young animals.

In accordance with the discoveries described above disclosed is that the Wnt signaling pathway is monitored or manipulated by measuring or modulating expression of one or more genes involved in the pathway, which the inventors have discovered are down-regulated in aged heart tissue. Such genes include Dlghl, Magi3, Akt1, Dab2, Rac1, Ctnnb1, Camk2d, Mapk1, Senp2, Smad3, Mark2, Ccnd1 and Pias4. Polynucleotides and fragments thereof that form these genes, as well as their encoded proteins and fragments, can be used, for example, in diagnostic or prognostic assays to measure the biological age of heart cells or tissues, or assays useful for screening test compounds for their effectiveness to retard heart aging, or in the manipulation of Wnt signaling in the heart to retard heart aging.

Disclosed is that the expression of at least one differentially expressed gene may be measured. Disclosed is that expression of two or more differentially expressed genes may be measured, providing a gene expression pattern or gene expression profile.

Disclosed is that changes in gene expression may be measured in one or both of two ways: (1) measuring transcription through detection of mRNA produced by a particular gene; and (2) measuring translation through detection of protein produced by a particular transcript.

Decreased or increased expression can be measured at the RNA level using any of the methods well known in the art for the quantitation of polynucleotides, such as, for example, PCR (including, without limitation, RT-PCR and qPCR), RNase protection, Northern blotting, microarray, macroarray, and other hybridization methods. The genes that are assayed or interrogated according to the present invention are typically in the form of mRNA or reverse transcribed mRNA. The genes may be cloned and/or amplified. The cloning itself does not appear to bias the representation of genes within a population. However, it may be preferable to use polyA+ RNA as a source, as it can be used with fewer processing steps.

Thus, disclosed is a combination comprising a plurality of polynucleotides or proteins expressed therefrom that are differentially expressed in heart tissue in aged subjects as compared with young subjects, wherein the polynucleotides are selected from two or more genes involved in Wnt signaling in the heart tissue, or fragments thereof. Disclosed is that the genes are selected from Dlgh1, Magi3, Akt1, Dab2, Rac1, Ctnnb1, Camk2d, Mapk1, Senp2, Smad3, Mark2, Ccnd1 and Pias4. Disclosed is that the genes are selected from Dlgh1, Magi3, Ctnnb1, Camk2d, Mapk1 and Senp2. Disclosed is that the genes are Magi3, Ctnnb1 and Camk2d. Disclosed is that the differential expression is reversed by CR or by ingestion of resveratrol, which may be administered as part of a dietary regimen, *e.g*., as an additive to food or feed, or as a dietary supplement.

Disclosed is that the combination comprises two or more polynucleotides or proteins expressed from the polynucleotides. Preferably, the combination comprises a plurality of polynucleotides or proteins expressed from polynucleotides, and may comprise up to all of the polynucleotides or proteins representing all of the genes listed above, or fragments thereof. When the combination comprises one or more fragments, the fragments can be of any size that retains the properties and function of the original polynucleotide or protein, preferably from about 30%, 60%, or 90% of the original.

The polynucleotides and proteins can be from any animal; for instance, humans or companion animals, such as dogs or cats. Homologs of the polynucleotides and proteins from different animal species are obtainable by standard information mining and molecular methods well known to the skilled artisan. For example, the name, or description of function of a gene or protein may be entered into one of several publicly available databases, which will generate a list of sources providing information about that gene from different species, including sequence information. One such database is the "Information Hyperlinked over Proteins (iHOP) database, which is accessible on the internet via the url: ihop-net.org. Alternatively, a public database accession number of a known gene or protein may be utilized to access sequence information for that gene or protein and to search for homologs or orthologs in other species using a sequence comparison search. For example, the GenBank accession number of a gene or protein from mouse may be entered into the National Institutes of Health's National Center for Biotechnology Information (NCBI) database, thereby accessing DNA or polypeptide sequences for that mouse gene. Using the same database, a BLAST search may be performed on the mouse DNA or protein sequence, or fragments thereof of sufficient length to define the gene or protein, to identify sequences of sufficient homology from other species, *e.g.*, a canine. Accession numbers of the sequences from the other species of interest may then be entered into the database to obtain information pertaining to those full-length nucleotide or protein sequences, as well as other descriptive information.

Disclosed is a composition comprising probes for detecting differential gene expression in heart tissue in aged subjects as compared with young subjects, wherein the probes detect two or more genes or gene products involved in Wnt signaling in the heart tissue. Disclosed is that the genes are selected from Dlgh1, Magi3, Akt1, Dab2, Rac1, Ctnnb1, Camk2d, Mapk1, Senp2, Smad3, Mark2, Ccnd1 and Pias4. Disclosed is that the genes are selected from Dlgh1, Magi3, Ctnnb1, Camk2d, Mapk1 and Senp2. Disclosed is that the genes are Magi3, Ctnnb1 and Camk2d. Disclosed is that the differential expression is reversed by CR or by ingestion of resveratrol, which may be administered as part of a dietary regimen, *e.g*., as an additive to food or feed, or as a dietary supplement.

The probes may comprise polynucleotides or oligonucleotides that specifically hybridize with the Wnt-related genes, or fragments thereof. Alternatively, they may comprise polypeptide binding agents that specifically bind to polypeptides produced by expression of the Wnt-related genes, or fragments thereof. Disclosed is that the polypeptide binding agents are antibodies, and also disclosed is that they are monoclonal antibodies. Disclosed is that the probes specifically hybridize or bind to human, canine or feline polynucleotides or polypeptides.

Typically, the composition comprises a plurality of probes, generally about 5, 10, 15,20, 25, 30, 35, 40, 45, 50, 60, 70, 80, 90, 100, or more probes for detecting polynucleotides or proteins, or fragments thereof, as appropriate for a particular species and use. It will be understood by the skilled artisan that multiple different probes for a single target gene or protein may be utilized, to refine the sensitivity or accuracy of an assay utilizing the probes. For example, several oligonucleotide probes, specifically hybridizing to different sequences on a target polynucleotide, may be employed. Likewise, several antibodies, immunologically specific for different epitopes on a target protein, may be utilized.

One or more oligonucleotide or polynucleotide probes for interrogating a sample may be prepared using the sequence information for any of the genes listed herein, from any species, such as human, canine or feline. The probes should be of sufficient length to specifically hybridize substantially exclusively with appropriate complementary genes or transcripts. Disclosed is that the oligonucleotide probes will be at least about 10, 12, 14, 16, 18, 20 or 25 nucleotides in length. Disclosed are longer probes of at least about 30, 40, 50, 60, 70, 80, 90 or 100 nucleotides are desirable, and probes longer than about 100 nucleotides may be suitable. The probes may comprise full length sequences encoding functional proteins. The nucleic acid probes are made or obtained using methods known to skilled artisans, *e.g., in vitro* synthesis from nucleotides, isolation and purification from natural sources, or enzymatic cleavage of the polynucleotides disclosed.

Hybridization complexes comprising nucleic acid probes hybridized to a polynucleotide comprising a Wnt-related gene may be detected by a variety of methods known in the art. Disclosed is that immobilized nucleic acid probes may be used for the rapid and specific detection of polynucleotides and their expression patterns. Typically, a nucleic acid probe is linked to a solid support and a target polynucleotide (*e.g.*, a gene, a transcription product, an amplicon, or, most commonly, an amplified mixture) is hybridized to the probe. Either the probe, or the target, or both, can be labeled, typically with a fluorophore or other tag, such as streptavidin. Where the target is labeled, hybridization may be detected by detecting bound fluorescence. Where the probe is labeled, hybridization is typically detected by quenching of the label. Where both the probe and the target are labeled, detection of hybridization is typically performed by monitoring a color shift resulting from proximity of the two bound labels. A variety of labeling strategies, labels, and the like, particularly for fluorescent based applications, are known in the art.

Disclosed is that the probes comprise polypeptide binding agents that specifically bind to polypeptides, or fragments thereof, produced by expression of one or more of the genes listed herein, or fragments thereof. Such protein binding probes may be prepared using the sequence information available for any of the Wnt-related proteins identified herein.

Assay techniques that can be used to determine levels of a protein in a sample are also well known to those of skill in the art. Such assay methods include radioimmunoassays, competitive-binding assays, Western blot analysis and ELISA assays. In the assay methods utilizing antibodies, both polyclonal and monoclonal antibodies are suitable for use in the invention. Such antibodies may be immunologically specific for a particular protein, or an epitope of the protein, or a protein fragment, as would be well understood by those of skill in the art. Methods of making polyclonal and monoclonal antibodies immunologically specific for a protein or peptide are also well known in the art.

Disclosed are antibodies for the detection and quantification of proteins produced by expression of the genes described herein. Though proteins may be detected by immunoprecipitation, affinity separation, Western blot analysis and the like, a preferred method utilizes ELISA-type methodology wherein the antibody is immobilized on a solid support and a target protein or peptide is exposed to the immobilized antibody. Either the probe, or the target, or both, can be labeled. A variety of labeling strategies, labels, and the like, are known in the art.

Disclosed is that arrays of oligonucleotide or polynucleotide probes may be utilized, and that arrays of antibodies or other proteins that bind specifically to the differentially expressed gene products. Such arrays may be custom made according to known methods, such as, for example, *in-situ* synthesis on a solid support or attachment of pre-synthesized probes to a solid support *via* micro-printing techniques. Disclosed is that arrays of nucleic acid or protein-binding probes are custom made to specifically detect transcripts or proteins produced by two or more of the differentially expressed genes or gene fragments described herein.

Disclosed are assay methods that involve monitoring changes in gene expression. Disclosed is that a method is provided for measuring a relative biological age of heart cells or tissue. The method comprises the steps of: (a) determining a test gene expression profile by measuring the transcription or translation products of one or more genes related to Wnt signaling in the heart cells or tissue; (b) comparing the test gene expression profile with the reference gene expression profile from equivalent heart cells or tissue of known biological age; and (c) determining from the comparison the relative biological age of the heart cells or tissue.

Disclosed is that the Wnt-related genes are Dlgh1, Magi3, Akt1, Dab2, Rac1, Ctnnb1, Camk2d, Mapk1, Senp2, Smad3, Mark2, Ccnd1 or Pias4. Disclosed is to utilize Dlghl, Magi3, Ctnnb1, Camk2d, Mapk1 or Senp2. Disclosed is to utilize Magi3, Ctnnb1 or Camk2d. Disclosed is that the expression of a gene encoding beta-catenin, *e.g*., Ctnnb1, is measured.

Disclosed is that the method is performed on a population of cultured cells, as described in greater detail in sections below. More typically, the method is performed on cells or tissue from an animal. Disclosed is that the method is uses to measure biological heart age by detecting differential expression of Wnt-related genes from humans or companion animals, most typically dogs or cats. Disclosed is that the probes are bound to a substrate, preferably in an array.

Disclosed is that the comparison between the test gene expression profile and the reference profile is relatively contemporaneous (*i.e*., heart cells or tissue from old and young subjects) is performed. Disclosed is that the reference used for comparison is based upon data previously obtained using the method. Disclosed is that the probes are exposed to a sample to form hybridization or binding complexes that are detected and compared with those of a standard, which may comprise previously gathered information correlating Wnt gene expression levels with a known biological age of the heart cells or tissue. The differences between the hybridization or binding complexes from the sample and standard indicate differential expression of polynucleotides and therefore genes differentially expressed in tissue of the old subject versus the standard, which can comprise mRNA previously isolated from a young subject or another type of reference subject.

Methods such as those described above may be useful for implementing, facilitating, or guiding an anti-aging regimen, such as CR and/or a nutritional regimen, or an exercise regimen. Such methods comprise obtaining a sample of heart tissue from a subject undergoing such a regimen. The tissue sample is then analyzed for modulated expression of one or more Wnt-related genes, using a gene or protein array or other detection method as described herein. The results of the analysis will reveal whether the treatment or regimen is effective in retarding heart aging in the individual.

In one aspect the invention provides a method for screening an agent or regimen for the ability to retard heart aging. This method comprises the steps of: (a) determining a first gene expression profile by measuring transcription or translation products of one or more genes related to Wnt signaling in heart tissue from an aged subject in the absence of the agent or regimen; (b) determining a second gene expression profile by measuring transcription or translation products of one or more genes related to Wnt signaling in heart tissue from an aged subject in the presence of the agent or regimen; and (c) comparing the first gene expression profile with the second gene expression profile, wherein a change in the second gene expression profile indicates that the agent or regimen is likely to be useful in retarding heart aging when administered to an individual.

Methods of this type may further comprise comparing at least the second gene expression profile with a reference gene expression profile obtained by measuring transcription or translation products of one or more genes related to Wnt signaling in heart tissue from a young subject or in heart tissue from an aged subject in the presence of a reference substance or regimen known to retard heart aging when administered to an individual. Such regimens or substances include, for instance, Caloric Restrion (CR) or ingestion of resveratrol.

Similar to the previous methods, the comparison between the test gene expression profile and the reference profile is relatively contemporaneous, *i.e.,* heart cells or tissue from old and young subjects are assayed side by side. Alternatively, however, the reference used for comparison can be based upon data previously obtained using the method. In this embodiment, the probes are exposed to a sample to form hybridization or binding complexes that are detected and compared with those of a standard, which may comprise previously gathered information correlating Wnt gene expression levels with a known biological age of the heart cells or tissue, or subjected to a reference regimen or treatment having a known effect on heart aging, as measured by Wnt-related gene expression (*e.g*., CR or ingestion of resveratrol).

In certain embodiments, the Wnt-related genes are Dlghl, Magi3, Akt1, Dab2, Rac1, Ctnnb1, Camk2d, Mapk1, Senp2, Smad3, Mark2, Ccnd1 or Pias4. Other embodiments utilize Dlgh1, Magi3, Ctnnb1, Camk2d, Mapk1 or Senp2. Still other embodiments utilize Magi3, Ctnnb1 or Camk2d. In a specific embodiment, expression of a gene encoding beta-catenin, e.g., Ctnnb1, is measured.

In one embodiment, the method is performed on a population of cultured cells. A nucleic acid construct comprising an Wnt-related gene according to the invention is introduced into cultured host cells. The host cells can be mammalian cell lines, preferably of cardiac origin or lineage, or progenitor lines known to differentiate into cardiac cells, as would be known in the art. The coding sequences of the genes are operably linked to appropriate regulatory expression elements suitable for the particular host cell to be utilized. The nucleic acid constructs can be introduced into the host cells according to any acceptable means in the art, including but not limited to, transfection, transformation, calcium phosphate precipitation, electroporation and lipofection. Such techniques are well known and routine in the art.

Gene expression assays can be carried out using a gene construct comprising the promoter of a selected aging-related gene operably linked to a reporter gene. The reporter construct may be introduced into a suitable cultured cell, including, without limitation, the standard host cell lines described above, or cells freshly isolated from a subject, such as adipose or muscle cells. The assay is performed by monitoring expression of the reporter gene in the presence or absence of a test compound.

In another embodiment, the method is performed on animals. Typically, a test compound or test regimen (dietary, exercise, and the like) is administered to a subject and the gene expression profile in a selected heart tissue of the subject is analyzed to determine the effect of the test compound on transcription or the translation of the Wnt-related genes or gene products. Gene expression can be analyzed *in situ* or *ex vivo* to determine the effect of the test compound. Disclosed is that a test compound or regimen is administered to a subject and the activity of a protein expressed from a gene is analyzed *in situ* or *ex vivo* according to any means suitable in the art to determine the effect of the test compound on the activity of the proteins of interest. In addition, where a test compound is administered to a subject, the physiological, systemic, and physical effects of the compound, as well as potential toxicity of the compound can also be evaluated.

Test substances can be any substance or combination of substances that may have an effect on heart aging as determined by a change, or reversal of an age-related change, in expression of Wnt-related genes as listed above. Suitable test substances include, but are not limited to, amino acids; proteins, peptides, polypeptides, nucleic acids, oligonucleotides, polynucleotides, small molecules, macromolecules, vitamins, minerals, simple sugars; complex sugars; polysaccharides; carbohydrates; medium-chain triglycerides (MCTs); triacylglycerides (TAGs); n-3 (omega-3) fatty acids including DHA, EPA, ALA; n-6 (omega-6) fatty acids including LA, γ-linolenic acid (GLA) and ARA; SA, conjugated linoleic acid (CLA); choline sources such as lecithin; fat-soluble vitamins including vitamin A and precursors thereof such as carotenoids (*e.g.*, (β-carotene), vitamin D sources such as vitamin D2 (ergocalciferol) and vitamin D3 (cholecalciferol), vitamin E sources such as tocopherols (*e.g*., α-tocopherol) and tocotrienols, as well as vitamin E derivatives such as trolox, and vitamin K sources such as vitamin K1 (phylloquinone) and vitamin K2 (menadione); water-soluble vitamins including B vitamins such as riboflavin, niacin (including nicotinamide and nicotinic acid), pyridoxine, pantothenic acid, folic acid, biotin and cobalamin; and vitamin C (ascorbic acid); antioxidants, including some of the vitamins listed above, especially vitamins E and C; also bioflavonoids such as apigenin, catechin, flavonone, genistein, naringenin, quercetin and theaflavin; quinones such as ubiquinone; carotenoids such as lycopene and lycoxanthin; and α-lipoic acid; L-carnitine; D-limonene; glucosamine; S-adenosylmethionine; chitosan; alginate; calcium; hyaluronic acid; magnesium; monooleylphosphatidic acid; nitric oxide (*e.g.,* as nitroglycerin); S-carbamylcysteine (an amino acid analog); sodium butyrate; sodium salicylate; spermidine; sphingosine; and glucose. Disclosed is that test substances are nutrients that may be added to food or consumed as a supplement.

Disclosed is a substance or regimen identified by the aforementioned method as likely to retard heart aging when administered to an individual. Due to the manner in which they are identified, such substances will increase Wnt signaling in the heart, or will increase expression or activity of one or more genes or gene products involved in Wnt signaling in the heart, or will at least partially reverse age-related decrease in Wnt-related gene expression or gene product activity in the heart.

Disclosed is a kit for measuring the biological age of heart cells or tissue. The kit comprises, in separate containers in a single package, or in separate containers in a virtual package: (1) reagents suitable for measuring expression of one or more genes related to Wnt signaling in the heart cells or tissue, and (2) instructions for how to use the measurements of expression of the genes related to Wnt signaling in the heart cells or tissue to determine the biological age of the heart cells or tissue. Discloed is that the kit comprises reagents suitable for measuring two or more Wnt-related genes. Disclosed is that the Wnt-related genes are Dlgh1, Magi3, Akt1, Dab2, Rac1, Ctnnb1, Camk2d, Mapk1, Senp2, Smad3, Mark2, Ccnd1 or Pias4. Disclosed is to utilize Dlgh1, Magi3, Ctnnb1, Camk2d, Mapk1 or Senp2. Disclosed is to utilize Magi3, Ctnnb1 or Camk2d. Disclosed is the expression of a gene encoding beta-catenin, e.g., Ctnnb1, is measured. Disclosed is that the amount or activity of beta-catenin in a sample is measured.

Disclosed is that the at least some of the reagents are probes comprising: (a) polynucleotides that specifically hybridize to or amplify transcription products of gene expression, or fragments thereof; or (b) polypeptide binding agents that specifically bind to translation products of gene expression, or fragments thereof. Disclosed is that the polypeptide binding agents are antibodies, which can be polyclonal and/or monoclonal antibodies. Disclosed is that the probes are bound to a substrate, and may be organized on the substrate in an array.

The kit may further comprise a reference for correlating the level of expression of the genes with heart aging. Such a reference may comprise one or more of (1) heart cells or tissues of one or more known biological ages, or (2) information communicating the level of expression expected for each gene in heart cells or tissues of one or more known biological ages. The kit may also comprise a reference substance, such as resveratrol, the ingestion of which is has been shown by the inventors to reverse the effect of heart aging on Wnt gene expression.

In any of the aforementioned types of kits, when the kit comprises a virtual package, the kit is limited to instructions in a virtual environment in combination with one or more physical kit components. Disclosed is that the kit contains probes and/or other physical components and the instructions for using the probes and other components are available via the internet. The kit may contain additional items such as a device for mixing samples, probes, and reagents and devices for using the kit, e.g., test tubes or mixing utensils.

Disclosed is a package comprising reagents suitable for measuring gene expression of one or more genes related to Wnt signaling in heart cells or tissue; the package further comprising a label affixed to the package, the label containing a word or words, picture, design, acronym, slogan, phrase, or other device, or combination thereof that indicates that the contents of the package contains reagents for determining the biological age of heart cells or tissue.

Disclosed is a computer system comprising a database containing information identifying expression levels of one or more genes related to Wnt signaling in heart cells or tissues, which are decreased in hearts of aged as compared with young animals, and a user interface that enables a user to access or manipulate the information in the database. The database can contain information identifying the expression level of one or more genes involved in Wnt signaling, such as Dlgh1, Magi3, Akt1, Dab2, Rac1, Ctnnb1, Camk2d, Mapk1, Senp2, Smad3, Mark2, Ccnd1 or Pias4, and a user interface to interact with the database, particularly to input, manipulate, and review the information for different animals or categories of animals. Disclosed is that the database further contains information identifying the activity level of one or more polypeptides encoded by Wnt-related genes. Disclosed is that the database further comprises sequence information for one or more of Wnt-related genes and their encoded proteins, preferably from a variety of species. Disclosed is that the database contains additional information pertaining to the description of the genes in one or more animal species. The computer system is any electronic device capable of containing and manipulating the data and interacting with a user, *e.g.,* a typical computer or an analytical instrument designed to facilitate using the invention and outputting the results relating to the status of an animal.

Disclosed is a medium for communicating information about, or instruction for use of one or more of (1) genes related to Wnt signaling in heart cells or tissues, which are decreased in hearts of aged as compared with young animals, (2) retarding heart aging in an individual by increasing Wnt signaling and/or increasing the amount or activity of beta-catenin in the individual's heart, (3) retarding heart aging in an individual by administering an agent or a regimen that increases Wnt signaling, and/or increases the amount or activity of beta-catenin in the individual's heart, (4) screening test compounds or regimens for their ability to retard heart aging by modulating expression of genes related to Wnt signaling in the heart, (5) determining the biological age of heart cells and tissue by measuring expression of genes related to Wnt signaling in the heart cells and tissues, and (6) kits and reagents for measuring differential expression of genes related to Wnt signaling in heart cells or tissues of aged as compared with young animals; wherein the medium comprises one or more of a physical or electronic document, digital storage media, optical storage media, audio presentation, audiovisual display, or visual display containing the information or instructions.

The medium may be selected from various media known in the art, or any combination of such media, including but not limited to: a displayed website, a visual display kiosk, a brochure, a product label, a package insert, an advertisement, a handout, a public announcement, an audiotape, a videotape, a DVD, a CD-ROM, a computer readable chip, a computer readable card, a computer readable disk, a USB device, a FireWire device, and/or a computer memory.

Disclosed are methods to retard heart aging in an individual, which are focused on increasing Wnt signaling in the individual's heart. The individuals may be animals of any species or kind that are aged, or susceptible to or suffering from a disease or condition that causes heart aging, including animals of any age, species, health condition, and the like. Preferably, the animals are humans or companion animals, such as dogs or cats. Disclosed is that the animals are aging animals susceptible to or suffering from aging of various cells and tissues of the heart. Disclosed is that the animals are animals susceptible to or suffering from a disease or condition that stresses or ages the heart, or a disease or condition of the heart that is associated with older individuals and that causes deleterious effects in other parts of the body. In either case, application of the methods of the invention to retard heart aging would have a beneficial effect on the individual's heart and thereby on the entire individual. Such conditions or diseases include, but are not limited to: cancer, AIDS, congestive heart disease, chronic obstructive pulmonary disease, renal failure, severe burns, heart attack (ischemia), coronary artery disease, atherosclerosis, surgery, e.g., angioplasty or heart bypass, valvular heart disease, hypertrophic cardiomyopathy, and the like. An individual may be deemed "susceptible" to heart aging or to the development of diseases or conditions associated with heart aging if the individual exhibits one or more risk factors for heart disease, blood vessel disease, and the like. Such risk factors are known to the skilled artisan, and include, but are not limited to: overweight, physical inactivity, high blood pressure, high cholesterol and/or triglycerides, angina pectoris, diabetes, smoking, heredity, being of the male sex, stress, and excessive alcohol ingestion.

A typical method for retarding heart aging in an individual comprises the steps of: (a) identifying an individual in which retarding heart aging is desired; and (b) modulating the expression of at least one gene that affects Wnt signaling in the individual's heart, wherein the modulating results in an increase of the Wnt signaling in the heart, thereby retarding the heart aging in the individual. Disclosed is that the Wnt-related genes are Dlgh1, Magi3, Akt1, Dab2, Rac1, Ctnnb1, Camk2d, Mapk1, Senp2, Smad3, Mark2, Ccnd1 or Pias4. Disclosed is the modulation of Dlghl, Magi3, Ctnnb1, Camk2d, Mapk1 or Senp2. Disclosed is to utilize Magi3, Ctnnb1 or Camk2d. Disclosed is the expression of a gene encoding beta-catenin, *e.g.,* Ctnnb1, is modulated. Disclosed is that the amount or activity of beta-catenin in an individual is modulated.

Disclosed is that the modulation comprises increasing expression of the one or more genes. Disclosed is that the increased Wnt signaling is associated with an increase in amount or activity of beta-catenin in the individual's heart.

Disclosed is administering to the individual an effective amount of one or more agents, or subjecting the individual to one or more regimens that increases Wnt signaling in the heart, or that at least partially reverses age-related decreases in Wnt signaling in the heart, thereby retarding the heart aging in the individual. The agent may act by mimicking the activity of beta-catenin in the individual's heart.

Disclosed is that regimens that include CR or ingestion of resveratrol may be utilized to retard heart aging by increasing Wnt signaling in the individual's heart or by at least partially reversing age-related decreases in Wnt signaling in the heart. Such regimens may be combined with other regimens or agents, such as those identified by the methods described herein.

Disclosed is that orally administering an individual, on a regular basis, preferably on an extended regular basis, a substance that increases Wnt signaling in the heart, thereby retarding heart aging. Typically the substance is regularly administered for at least two weeks, more preferably for at least four weeks or longer. Administration of the substance may continue indefinitely, *e.g*., for one, two, three, six or nine months, or for a year or more, or even for the life of the individual. The substance generally is administered at least daily, but the dosage regimen will depend on the nature and potency of the substance. Accordingly, administration may be more frequent, *e.g.,* twice or three times daily, or less frequent, *e.g.,* three times weekly, twice weekly, weekly, twice monthly or monthly.

Disclosed is that the extended regular oral administration of the substance causes a change in expression, or a reversal of an age-related change in expression, of one or more of Dlgh1, Magi3, Akt1, Dab2, Rac1, Ctnnb1, Camk2d, Mapk1, Senp2, Smad3, Mark2, Ccnd1 or Pias4. Disclosed is to focus on the modulation of Dlgh1, Magi3, Ctnnb1, Camk2d, Mapk1 or Senp2. Disclosed is to utilize Magi3, Ctnnb1 or Camk2d. Disclosed is the expression of a gene encoding beta-catenin, *e.g.*, Ctnnb1, is modulated by the extended regular oral administration of the substance. Disclosed is that the amount or activity of beta-catenin in an individual is modulated. Resveratrol is an example of a substance that, when orally administered to a subject on an extended regular basis, reverses age-related changes in Wnt-related gene expression in the heart.

Disclosed is that the modulation comprises increasing expression of the one or more genes. Disclosed is that the increased Wnt signaling is associated with an increase in amount or activity of beta-catenin in the individual's heart.

When utilized as a supplement to ordinary dietetic requirements, the substance may be administered directly to the animal. The substance can alternatively be contacted with, or admixed with, daily feed or food, including a fluid, such as drinking water, or supplied as a dietary supplement. When utilized in conjunction with or incorporated into a daily feed or food, administration will be well known to those of ordinary skill. Administration can be carried out as part of a dietary regimen for the animal. For example, a dietary regimen may comprise causing the regular ingestion by the animal of the substance, in an amount effective to retard heart aging. Disclosed is that the substance is administered to the animal in conjunction with one or more drugs, nutraceuticals, or nutritional agents for retarding heart aging or for increasing longevity generally.

Disclosed is that daily or periodic doses for the substance administered in accordance with this method range from about 0.001 g/kg body weight to 10 g/kg body weight. More particularly, the dose exceeds 0.01, 0.02, 0.03, 0.04, 0.05, 0.06, 0.07, 0.08, 0.09 or 0.1 g/kg body weight. Disclosed is that the dosage may be 0.2, 0.5, 1, 3, 5, 7, or 10 g/kg body weight or more, depending on the substance and dosing frequency. The skilled artisan is familiar with the development of dosages and dosing regimens for subjects.

### EXAMPLES

The invention can be further illustrated by the following example, although it will be understood that this example is included merely for purposes of illustration and are not intended to limit the scope of the invention unless otherwise specifically indicated.

### Example 1

### Materials and methods

Two public rodent cardiac aging gene expression data sets were selected from the NCBI's Gene Expression Omnibus (GEO): (1) The University of Washington's rat study (UW) (Linford JL et al. (2007) Transcriptional Response to Aging and Calorie Restriction in Heart and Adipose Tissue. Aging Cell 6: 673-688)) (Study 1); and (2) LifeGen's mouse study (Barger JL et al. (2008) A Low Dose of Dietary Resveratrol partially Mimics Calorie Restriction and Retards Aging Parameters in Mice. PLoS ONE 3(6): 1-10) (Study 2). Materials and methods for the two studies are shown in the references. A third data set came from an unpublished mouse cardiac aging study. In that study, mice were divided into two groups (n=7) and were fed with control diet according to the proceedings in the Barger study. Mice were sacrificed at the ages of 5 months (young group) and 25 months (old group). Heart tissues were collected and subject to microarray gene expression assays (Study 3). In Study 2 and Study 3, Affymetrix mouse genome 430 2.0 gene chips were used. In Study 1, Affymetrix rat genome 230 2.0 gene chips were used. A description of the three studies is outlined in Table 1.

**Table 1**

| | | | Design | | | | |
|---|---|---|---|---|---|---|---|
| Name | Source | Species | Young | Old | CR | Resveratrol | Sample # |
| Study 2 | NCBI | Mouse | 5 | 30 | 30+CR | 30+Resveratrol | 5 |
| Study 1 | NCBI | Rat | 4 | 28 | 28+CR | / | 6 |
| Study 3 | Internal | Mouse | 5 | 25 | / | / | 7 |

Data were first inspected for quality and examined for possible outliers. Three samples, one from each Study, were identified as outliers and were removed from further analysis. Differential genes were identified between young and old groups in each Study using Significance Analysis of Microarrays (SAM) algorithm (Tusher, Tibshirani and Chu (2001) Significance analysis of microarrays applied to the ionizing radiation response. PNAS 2001 98: 5116-5121). The selection criteria include magnitude of expression change (fold>=1.2) and false discovery rate (FDR<0.5%).

To determine the impact of gene expression changes in the systems level, pathway analysis was conducted on the data sets from Study 2 and Study 3 using GenMAPP and MAPPFinder software (Salomonis, K Hanspers, AC Zambon, K Vranizan, SC Lawlor, KD Dahlquist, SW Doniger, J Stuart, BR Conklin, and AR Pico. GenMAPP 2: new features and resources for pathway analysis. BMC Bioinformatics, Jun 2007; 8: 217). Probes from the entire chip were used with the same selection criteria, fold>1.2 and FDR<0.5%. Pathways with the most significant associations within the data were selected. The inventors determined that the Wnt signaling pathway was among those with most significant associations (adjusted p value of 0.011 in both the Study 2 and Study 3 data sets).

Genes in the Wnt pathway were further examined for their expressional changes. About a dozen genes showed decreased expressions in aged heart compared with young heart in Study 2. Those genes/proteins are listed in Table 2, along with respective representative GenBank Accession Numbers, and the Study 2 results are shown in Table 3. The expression of these genes was also examined in the calorie restricted (CR) group and resveratrol ("Resv") group. As shown in Table 3, the expression of all but three these genes was elevated to their young levels with CR or Resv treatment. The majority of the genes down-regulated in aged heart were positive determinants of the Wnt pathway, indicating that the Wnt pathway is down-regulated in old versus young hearts in mice.

**Table 2**

| Genes Differentially Regulated in Old Versus Young Heart Tissue (Study 2) | | | | | |
|---|---|---|---|---|---|
| | | GenBank Accession No. | | Protein Accession No. | |
| Probe ID | Symbol | Mouse | Rat | Mouse | Rat |
| 1450768_at | Dlgh1 | NM_007862 | NM_012788 | NP_031888 | NP_036920 |
| 1421035_a_at | Magi3 | NM_001159354 | NM_139084 | NP_001152826 | NP_620784 |
| 1425711_a_at | Akt1 | NM_009652 | NM_033230 | NP_033782 | NP_150233 |
| 1430604_a_at | Dab2 | NM_023118 | NM_024159 | NP_075607 | NP_077073 |
| 1423734_at | Rac1 | NM_009007 | NM_134366 | NP_033033 | NP_599193 |
| 1430533_a_at | Ctnnb 1 | NM_001165902 | NM_053357 | NP_001159374 | NP_445809 |
| 1450008_a_at | Ctnnb1 | NM_001165902 | NM_053357 | NP_001159374 | NP_445809 |
| 1427763_a_at | Camk2d | NM_001025438 | NM_012519 | NP_001020609 | NP_036651 |
| 1426585_s_at | Mapk1 | NM_001038663 | NM_053842 | NP_001033752 | NP_446294 |
| 1425465_a_at | Senp2 | NM_029457 | NM_023989 | NP_083733 | NP_076479 |
| 1450471_at | Smad3 | NM_016769 | NM_013095 | NP_058049 | NP_037227 |
| 1435889_at | Mark2 | NM_001080388 | NM_021699 | NP_001073857 | NP_067731 |
| 1448698_at | Ccnd1 | NM_007631 | NM_171992 | NP_031657 | NP_741989 |
| 1455394_at | Pias4 | NM_021501 | NM_001100757 | NP_067476 | NP_001094227 |

**Table 3**

| Change in Wnt Pathway Gene Expression in Old versus Young Heart Tissue, and as Affected by CR or Resveratrol (Study 2) | | | | | | |
|---|---|---|---|---|---|---|
| | (A) Fold Change* | | | (B) FDR | | |
| Symbol | Old vs.Yng | CR vs. Old | Resv vs. Old | Old vs.Yng | CR vs. Old | Resv vs. Old |
| Dlgh1 | -1.528 | 1.154 | 1.150 | 0.010 | NS** | NS |
| Magi3 | -1.366 | 1.195 | 1.244 | 0.010 | 0.010 | 0.050 |
| Akt1 | -1.814 | -1.119 | -1.315 | 0.010 | NS | NS |
| Dab2 | -1.296 | 1.201 | 1.227 | 0.010 | 0.010 | 0.010 |
| Rac1 | -2.004 | 1.712 | 1.509 | 0.010 | 0.010 | 0.010 |
| Ctnnb1(1) | -3.038 | 3.177 | 2.787 | 0.010 | 0.010 | 0.010 |
| Ctnnb1(2) | -1.904 | 2.143 | 2.017 | 0.010 | 0.010 | 0.010 |
| Camk2d | -1.933 | 1.541 | 1.235 | 0.010 | 0.010 | 0.050 |
| Mapk1 | -1.290 | -1.035 | -1.020 | 0.010 | NS | NS |
| Senp2 | -1.538 | 1.071 | 1.261 | 0.010 | NS | 0.050 |
| Smad3 | -1.371 | 1.412 | 1.285 | 0.010 | 0.010 | 0.050 |
| Mark2 | 1.531 | -2.069 | -2.113 | 0.010 | 0.010 | 0.010 |
| Ccnd1 | 1.395 | -1.651 | -1.158 | 0.010 | 0.010 | NS |
| Pias4 | 1.306 | -1.514 | -1.495 | 0.010 | 0.010 | 0.010 |

| | | | | | | |
|---|---|---|---|---|---|---|
| * Numbers indicate expression changes in fold (A) and False Discovery Rate (B) ** NS = not significant | | | | | | |

An integration study was conducted to identify genes with similar expression profile changes in all three studies and to identify potential biomarkers. The results are shown in Table 4. Three genes, Magi3, Ctnnb1, and Camk2d, were down-regulated in old heart vs. young heart in all three Studies. A fourth gene, Senp2, was down-regulated in two Studies (Study 1 and Study 2). Special attention was paid to beta-catenin (ctnnb1), a key component in Wnt signaling pathway. The beta-catenin plays an important role in the Wnt signal transduction pathway, as it mediates Wnt signal into the nucleus to partner with different transcription factors to activate an array of downstream genes. As shown in Table 4, beta-catenin is down-regulated up to three-fold (-3.04) in old heart in Study 2 and its expression returns to its young level with CR (3.18) or Resveratrol (2.79) supplemented diet. A second beta-catenin probe also exhibited similar change with slightly less magnitude. The expression fold change for beta-catenin was -1.3 and -1.5 in old versus young hearts in Study 3 and Study 1, respectively. Similar expression profile change was evident for Magi3 and Camk2d. In addition, two genes, Dlgh1 and Mapk1, were down-regulated in both Study 1 and Study 2. This results show that beta-catenin is a biomarker for cardiac aging.

**Table 4**

| Differential Expression of Wnt Pathway Genes in Three Gene Expression Profiles of Old versus Young Heart Tissue, and Treatment with CR or Resveratrol | | | | | |
|---|---|---|---|---|---|
| | Study 3 | Study 1 | Study 2 | | |
| Symbol | Old vs. Yng | Old vs.Yng | Old vs.Yng | CR vs. Old | Resv vs. Old |
| Dlgh1 | | -1.3 | -1.528 | | |
| Magi3 | -1.6 | -1.4 | -1.366 | 1.195 | 1.244 |
| Ctnnb1 probe 1 | -1.3 | -1.5 | -3.038 | 3.177 | 2.787 |
| Ctnnb1 Probe2 | | | -1.904 | 2.143 | 2.017 |
| Camk2d | -2.1 | -1.2 | -1.933 | 1.541 | 1.235 |
| Mapk1 | | -1.2 | -1.290 | | |
| Senp2 | 1.5 | -1.3 | -1.538 | 1.071 | 1.261 |

| | | | | | |
|---|---|---|---|---|---|
| Numbers indicate expression changes in fold | | | | | |

## Claims

1. A method for screening an agent or regimen for the ability to retard heart aging comprising the steps of:
a) determining a first gene expression profile by measuring transcription or translation products of one or more genes related to Wnt signaling in heart tissue from an aged subject in the absence of the agent or regimen;
b) determining a second gene expression profile by measuring transcription or translation products of one or more genes related to Wnt signaling in heart tissue from an aged subject in the presence of the agent or regimen; and
c) comparing the first gene expression profile with the second gene expression profile, wherein a change in the second gene expression profile indicates that the material or regimen is likely to be useful in retarding heart aging when administered to an individual.

2. The method of claim 1 further comprising comparing at least the second gene expression profile with a reference gene expression profile obtained by measuring transcription or translation products of one or more genes related to Wnt signaling in heart tissue from a young subject or in heart tissue from an aged subject in the presence of a reference substance or regimen known to retard heart aging when administered to an individual; optionally wherein the reference substance or regimen comprises caloric restriction or ingestion of resveratrol.

3. The method of claim 1 wherein the genes are Dlgh1 , Magi3, Akt1, Dab2, Rac1, Ctnnb1, Camk2d, Mapk1, Senp2, Smad3, Mark2, Ccnd1 or Pias4.

4. The method of claim 3 wherein the genes are Dlghl, Magi3, Ctnnb1, Camk2d, Mapk1 or Senp2.

5. The method of claim 4 wherein the genes are Magi3, Ctnnb1 or Camk2d.

6. The method of claim 5 wherein the gene is Ctnnb1.

## Patentansprüche

1. Verfahren zum Screenen eines Mittels oder Schemas auf die Fähigkeit, ein Altern des Herzens zu verzögern, die folgenden Schritte umfassend:
a) Bestimmen eines ersten Genexpressionsprofils durch Messen von Transkriptions- oder Translationsprodukten von einem oder mehreren Genen im Zusammenhang mit Wnt-Signalgebung in Herzgewebe von einer alten Person in Abwesenheit eines Mittels oder Schemas;
b) Bestimmen eines zweiten Genexpressionsprofils durch Messen von Transkriptions- oder Translationsprodukten von einem oder mehreren Genen im Zusammenhang mit Wnt-Signalgebung in Herzgewerbe von einer alten Person in Anwesenheit eines Mittels oder Schemas; und
c) Vergleichen des ersten Genexpressionsprofils mit dem zweiten Genexpressionsprofil, wobei eine Veränderung bei dem zweiten Genexpressionsprofil darauf hinweist, dass das Material oder das Schema bei Verabreichung an ein Individuum wahrscheinlich einen Nutzen bei der Verzögerung von Herzalterung aufweist.

2. Verfahren nach Anspruch 1, ferner umfassend das Vergleichen von mindestens dem zweiten Genexpressionsprofil mit einem Referenzgenexpressionsprofil, das durch Messen von Transkriptions- oder Translationprodukten von einem oder mehreren Genen im Zusammenhang mit Wnt-Signalgebung in Herzgewebe von einer jungen Person oder in Herzgewebe von einer alten Person in Anwesenheit einer Referenzsubstanz oder eines Referenzschemas, von der oder von dem bekannt ist, dass es bei Verabreichung an ein Individuum das Altern des Herzens verzögert, erhalten wurde; wahlweise wobei die Referenzsubstanz oder das Referenzschema eine kalorische Begrenzung oder Aufnahme von Resveratrol umfasst.

3. Verfahren nach Anspruch 1, wobei es sich bei den Genen um Dlgh1, Magi3, Akt1, Dab2, Rac1, Ctnnb1, Camk2d, Mapk1, Senp2, Smad3, Mark2, Ccnd1 oder Pias4 handelt.

4. Verfahren nach Anspruch 3, wobei es sich bei den Genen um Dlgh1, Magi3, Ctnnbl1, Camk2d, Mapk1, Senp2 handelt.

5. Verfahren nach Anspruch 4, wobei es sich bei den Genen um Magi3, Ctnnb1 oder Camk2d handelt.

6. Verfahren nach Anspruch 5, wobei es sich bei dem Gen um Ctnnb1 handelt.

## Revendications

1. Procédé de criblage d'un agent ou d'un régime pour déceler la capacité à retarder le vieillissement cardiaque, comprenant les étapes consistant à :
a) déterminer un premier profil d'expression génique en mesurant des produits de transcription ou de traduction d'un ou plusieurs gènes se rapportant à la signalisation de Wnt dans un tissu cardiaque provenant d'un sujet âgé en l'absence de l'agent ou du régime ;
b) déterminer un second profil d'expression génique en mesurant des produits de transcription ou de traduction d'un ou plusieurs gènes se rapportant à la signalisation de Wnt dans un tissu cardiaque provenant d'un sujet âgé en présence de l'agent ou du régime ; et
c) comparer le premier profil d'expression génique au second profil d'expression génique, dans lequel un changement dans le second profil d'expression génique indique que le matériau ou régime est susceptible d'être utile pour retarder le vieillissement cardiaque lorsqu'il est administré à un individu.

2. Procédé selon la revendication 1, comprenant en outre la comparaison d'au moins le second profil d'expression génique à un profil d'expression génique de référence obtenu en mesurant des produits de transcription ou traduction d'un ou plusieurs gènes se rapportant à la signalisation de Wnt dans un tissu cardiaque provenant d'un sujet jeune ou dans un tissu cardiaque provenant d'un sujet âgé en présence d'une substance ou d'un régime de référence connu pour retarder le vieillissement cardiaque lorsqu'il est administré à un individu ; éventuellement dans lequel la substance ou le régime de référence comprend une restriction calorique ou une ingestion de resvératrol.

3. Procédé selon la revendication 1, dans lequel les gènes sont Dlgh1, Magi3, Akt1, Dab2, Rac1, Ctnnb1, Camk2d, Mapk1, Senp2, Smad3, Mark2, Ccnd1 ou Pias4.

4. Procédé selon la revendication 3, dans lequel les gènes sont Dlgh1, Magi3, Ctnnb1, Camk2d, Mapk1 ou Senp2.

5. Procédé selon la revendication 4, dans lequel les gènes sont Magi3, Ctnnb1 ou Camk2d.

6. Procédé selon la revendication 5, dans lequel le gène est Ctnnb1.
